# EUROPEAN PATENT SPECIFICATION

(11) **EP 0 465 530 B1**
(45) Date of publication and mention of the grant of the patent: **05.05.1993**
(21) Application number: 90905284.7
(22) Date of filing: 19.03.1990
(51) Int. Cl.: A61F 5/44

(54) **MALE URINARY ANTI-INCONTINENCE DEVICE AND METHOD**
HARNINKONTINENZEINRICHTUNG FÜR MÄNNER UND VERFAHREN
PROCEDE ET DISPOSITIF CONTRE L'INCONTINENCE URINAIRE CHEZ L'HOMME

(30) Priority: 20.03.1989 US 325599
(43) Date of publication of application: 15.01.1992
(73) Proprietor: DACOMED CORPORATION, Minneapolis, MN 55425 (US)
(72) Inventor: ERICKSON, Richard, A., St. Paul, MN 55117 (US); TIMM, Gerald, W., Deephaven, MN 55331 (US)
(74) Representative: Ström, Tore
(86) International application number: US9001480
(87) International publication number: WO9011063

(56) References cited:
- US-A- 1 728 322
- US-A- 2 533 924
- US-A- 2 618 270
- US-A- 2 756 753
- US-A- 3 147 754
- US-A- 3 155 096
- US-A- 3 203 421
- US-A- 3 866 611
- US-A- 4 534 353
- US-A- 4 800 900
- William E. Bradley, M.D et al "Urinary Incontinence: Control by External Device", 1973, Arch.Phys.Med.Rehabil., Vol. 54 pp. 376-378
- Paul Citron et al "An external device for management of male urinary incontinence", 1972, Pergamon Press, Minneapolis, Vol. 5 pp. 257-260

## Description

### Technical Field of the Invention

The present invention relates to an external male urinary anti-incontinence device and method. In particular the invention relates to a device and method for preventing the involuntary leakage of urine in incontinent males by applying sufficient pressure to occlude the urethra along the ventral penile shaft.

### Background of the Invention

There are many types of devices and methods for treating male urinary incontinence using external penile appliances. A spring loaded, external penile device is discussed in two articles: See Urinary Incontinence: Control by External Device, Archives of Physical Medicine and Rehabilitation, August 1973, Vol. 54, pp. 376-378 and An External Device for Management of Male Urinary Incontinence, J. Biomechanics, 1972, Vol. 5, pp. 257-260.

At least four such devices are currently being marketed: 1) Cunningham Clamp - Bard Home Health Care Division, C.R. Bard, Inc., Berkley Heights, NJ 07922; 2) Baumrucker Clamp - Greenwald Surgical Co., Inc., 2688 DeKalb St., Lake Station, IN 46405; 3) Penoring Clamp - Koken Co., Ltd., Tokyo, Japan; and 4) Cook Continence Cuff - VPI, A Cook Group Company, 127 South Main Street, Spencer, IN 47460.

Known U.S. patents are 4,102,342; 3,866,611; 3,203;421; 3,155,096; 3,147,754; 2,756,753; 2,618,270; 2,533,924; and 1,728,322.

There are several disadvantages associated with many existing devices and methods for treating male urinary incontinence using an external penile appliance. One typical problem is that these devices often tend to restrict the blood supply to the penile shaft which can cause disconfort to the user and in extreme cases can result in tissue necrosis. This is due in part because many of these devices restrict the penile shaft between two relatively flat, narrow, and rigid members.

Yet another problem with many existing devices is that they orient the hinge axis parallel to the axis of the penile shaft. This often results in uneven pressure zones on the penile shaft. In particular, devices with a parallel hinge axis orientation typically apply pressure on the tissue positioned closest to the hinge.

Yet another problem with many existing devices is they are sensitive to tension adjustment such that the tension affects the force applied by the device to the penile shaft. Thus it is possible for the user to inadvertently tighten the device too much such that the blood supply to the penis is restricted.

Many existing devices are bulky and difficult for the user to conceal. Snaps, hooks, buckles, nonelastic tape, etc. which are used to attach the devices to the penile shaft are often uncomfortable and a source of irritation.

Additionally, the cost of existing devices is often too expensive to be disposable after a period of use or when becoming soiled. Failure to replace these devices because of soiling can result in reduced personal hygiene and patient safety.

The present invention solves these and other problems associated with the prior art.

### Summary of the Invention

The present invention relates to an external male anti-incontinence device including a cradle member having a dorsal arm member with a curvilinear surface and a ventral arm member with a curvilinear surface. The dorsal and ventral arm members are in axial alignment with each other with an aperture being defined intermediate of the dorsal and ventral arm members. The ventral arm member includes a pressure pad integral therewith. The cradle member includes integral hinges interconnecting the dorsal and ventral arm members, the cradle member being hinged about an axis perpendicular to the axial alignment of the dorsal and ventral arm members whereby the cradle member can be folded over into a folded state so that the curvilinear surfaces of the dorsal and ventral arm members are facing one another with the pressure pad projecting into a cavity formed between the facing curvilinear surfaces of the dorsal and ventral arm members. A strap assembly is used for releasably retaining the cradle member in the folded state.

The external male anti-incontinence device of the present invention incorporates a curvature in the dorsal and ventral arm members which conforms to the cylindrical contour of the penile shaft. This causes the force applied to the penile shaft by the invention to be applied radially and eliminates localized areas of constricted blood flow in the penile shaft. The dorsal and ventral arm members are sized and configured such that although the forces applied by the pressure pad, also referred to as occlusion pad, are sufficient to prevent urine leakage through the urethra, the overall pressure applied to the penile shaft is reduced due in part to the increased contact area of the dorsal and ventral arm members on the penile shaft.

The present invention has a hinge axis which is perpendicular to the penile shaft axis and to the axial alignment of the dorsal and ventral arm members which assists in alignment of the dorsal and ventral arm members and prevents the occurrence of any uneven pressure zones on the penile shaft.

One embodiment of the present invention includes cradle means for positioning on a penile shaft, the cradle means including a dorsal arm member and a ventral arm member in axial alignment with one another generally along a longitudinal axis of the cradle means. The cradle means further includes an aperture intermediate of the dorsal and ventral arm members. The cradle means is hinged about an axis perpendicular to the longitudinal axis of the cradle means so that the cradle means is foldable between an unfolded state and a folded state wherein the dorsal and ventral arm members are facing one another. The dorsal and ventral arm members are releasably connected for retaining the cradle means in the folded state.

The external male anti-incontinence device of the present invention is operated by inserting the penile shaft through the aperture intermediate of the dorsal and ventral arm members, hinging the device into a folded, operative state so that the pressure pad is blocking the urethra, and retaining the device in the folded state on the penile shaft with the releasable strap assembly.

A preferred embodiment of the present invention inherently limits the amount of pressure that can be applied to the penile shaft and provides the flexibility to accommodate changes in penile shaft size while wearing the device. This ability to accommodate changes in penile shaft size is due in large part to the use of an elastic strap assembly which attaches the device to the penile shaft.

In the preferred embodiment, the integral urethral occlusion pad (pressure pad) of the device limits the pressure applied to the urethra to a specific amount above the pressure applied to the penile shaft by the ventral and dorsal arm members. This is accomplished by limiting the extent that the occlusion pad protrudes from the concave surface of the ventral arm member. Additionally, this feature reduces the possibility of restricting the blood supply to the penile shaft and decreases the significance of tension in the strap assembly so as to assure proper attachment of the device to the penile shaft.

A preferred embodiment of the present invention includes flanges along the periphery of the dorsal and ventral arm members which prevent closure of the device beyond contact between the flanges. This limits the maximum force that can be applied to the penile shaft and the urethra.

An advantage of a preferred embodiment of the present invention is that the cradle member with its dorsal and ventral arm members, hinges, and pressure pad is made of a one- piece construction so as to reduce the manufacturing costs such that the device can be disposed of and replaced after a period of use or when the device has become soiled, thereby resulting in improved personal hygiene and patient safety.

Yet another advantage of a preferred embodiment of the present invention is that it is more compact and concealable than existing devices.

A preferred embodiment of the present invention is fabricated from a composite material made up of a semi-rigid polyolefin surface coating fused to closed cell, chemically cross-linked polyethylene foam. In the preferred embodiment, the composite material is thermoformed to the desired contour and then die cut to the correct external dimensions. In the preferred embodiment, the present invention is sufficiently sized to generally retain its shape and yet have some give and flexibility.

Another advantage of a preferred embodiment of the present invention is that it uses an elastic strap which increases user comfort and allows for variable separation of the dorsal and ventral arm members as the penile shaft increases and decreases in diameter.

These and various other advantages and features of novelty which characterize the invention are pointed out with particularity in the claims annexed hereto and forming a part hereof. However, for a better understanding of the invention, its advantages, and the objects obtained by its use, reference should be made to the accompanying drawings and descriptive matter, which form a further part hereof, and in which there is illustrated and described a preferred embodiment of the invention.

### Brief Description of the Drawings

In the drawings wherein like reference numerals generally indicate corresponding parts throughout the several views;

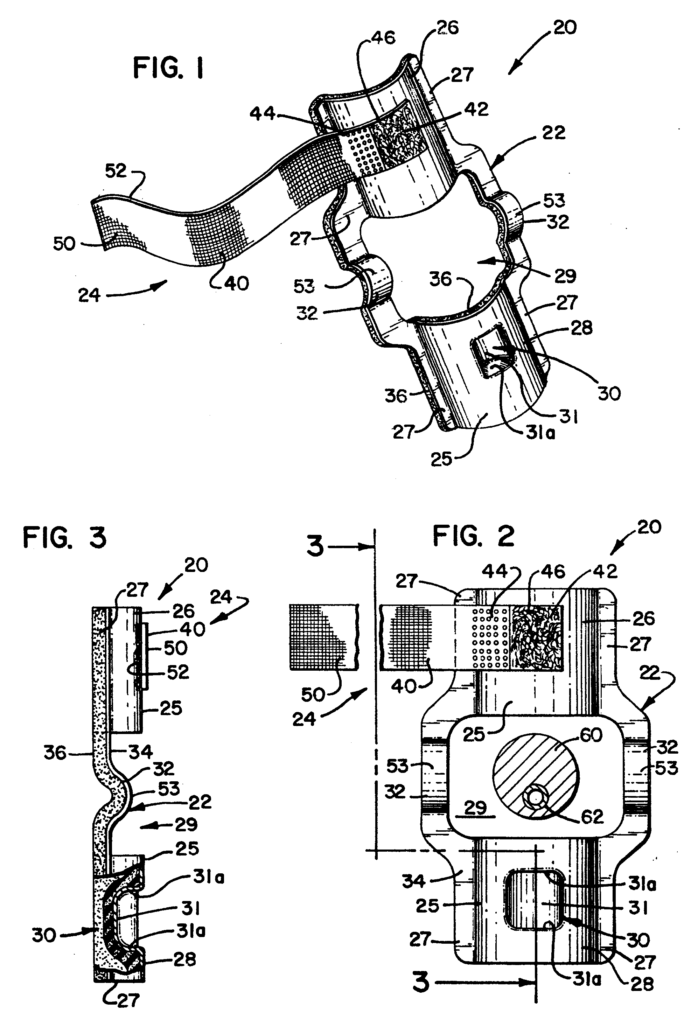

Figure 1 is a perspective view of a preferred embodiment of an external male anti-incontinence device in accordance with the principles of the present invention;
Figure 2 is a planar view of the embodiment shown in Figure 1;
Figure 3 is a sectional view as generally seen along line 3-3 in Figure 2;
Figure 4 is a perspective view illustrating placement of the device shown in Figure 1 onto a penis;
Figure 5 is a perspective view illustrating the device shown in Figure 1 in its folded, operative state on the penis;
Figure 6 is a sectional view as generally seen along line 6-6 in Figure 5 illustrating minimal spacing between the dorsal and ventral arm members;
Figure 7 is a view similar to Figure 4 illustrating an alternative method of placing the device onto a penis such that concave surfaces of the device face away from the user; and
Figure 8 is a view similar to Figure 5 showing the device in its folded, operative state after being inserted onto the penis according to the method shown in Figure 7.

### Detailed Description of a Preferred Embodiment of the Present Invention

Referring now to the figures, there is illustrated a preferred embodiment of an external male anti-incontinence device in accordance with the principles of the present invention, the device being referred to by the reference numeral 20. The device 20 includes a one-piece cradle member 22 and a strap assembly 24. The cradle member 22 includes a dorsal arm member 26 and a ventral arm member 28 in axial alignment with each other. The dorsal and ventral arm members 26, 28 have a curvilinear surface 25 with axially extending flanges 27 extending along the sides thereof. An aperture 29, sized to receive a penile shaft, is defined intermediate of the dorsal and ventral arm members 26, 28. The ventral arm member 28 includes integral therewith a urethral occlusion pad 30, also referred to as a pressure pad, having a bottom wall portion 31 and curvilinear side wall portions 31a. The cradle member 22 includes integral hinges 32 interconnecting the dorsal and ventral arm members 26, 28. The cradle member 22 is hinged about an axis perpendicular to the axial alignment of the dorsal and ventral arm members 26, 28 and to the longitudinal axis of the penile shaft when positioned thereon.

The cradle member 22 has a semi-rigid plastic coating layer 34 and a soft foam layer 36. The plastic layer 34 is disposed on convex side of the curvilinear surfaces 25 with the foam layer 36 disposed on a concave side of the curvilinear surfaces 25. In the preferred embodiment the cradle member 22 is fabricated from VOLEX (a U.S. registered trademark) extrusion coated composite made up of polyolefin surface coatings fused to closed-cell, chemically cross-linked polyethylene foam. VOLEX is available from Voltek, a Division of Sekisui America Corp., 100 Shepard Street, Lawrence, MA 01843. The material is thermoformed to the desired contour and then die cut to the correct external dimensions. Different sizes of the cradle member 22 are preferably made to fit different diameters of penile shafts.

The cradle member 22 can also be constructed using alternative materials and known fabrication techniques. Examples include, but are not limited to, thermoformed polystyrene with separately attached foam padding and injection molded or extruded plastics and/or nonplastics with separately attached or integral foam padding.

In the preferred embodiment shown, the strap assembly 24 includes an elastic strap 40 having an elastic backing 50 and a loop material 52. The elastic strap 40 is attached to a strap 42 proximate an end portion 44 by ultrasonic welding. The strap 42 is attached to the convex side of the dorsal arm member 26 by a pressure sensitive adhesive layer 46. The strap 42 includes hook material for releasable attachment to the loop material 52 of the elastic strap 40. In one embodiment of the present invention, the elastic strap 40 is a length of VEL-STRETCH (registered U.S. trademark) elasticized loop tape and the strap 42 is VELCRO (registered U.S. trademark) hook tape. The elasticized loop tape is ultrasonically welded to the hook tape in a back strap configuration. The elastic strap 40 accommodates changes in penile shaft size by allowing the dorsal and ventral arm members 26, 28 to move apart from one another as the penile shaft size increases. Then when the penile shaft size decreases, the elastic strap 40 pulls the dorsal and ventral arm members 26, 28 back toward each other.

The strap assembly can also be constructed using alternative materials including, but not limited to, nonelastic loop tape, elastic webbing, foam tape, and plastic or cloth materials. Closure methods could include, but are not limited to, snaps, hooks, buckles, and pressure sensitive adhesives.

In a preferred embodiment shown, the integral hinges 32 are formed in the cradle member 22 by raised arches 53 where the hinge action is to occur. The hinges are preferably formed when thermoforming the surface coating 34 and its associated foam layer 36 to the desired contour.

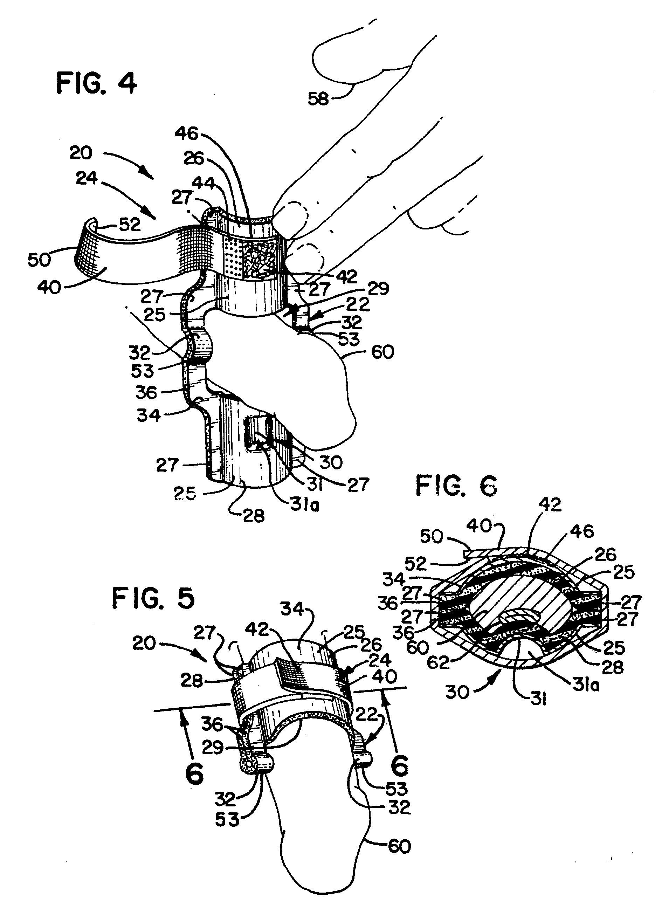

Referring now to Figures 4-6, the external male anti-incontinence device 20 is illustrated in Figure 4 while being positioned onto a penile shaft 60 and is illustrated in Figure 5 in its folded, operative state on the penile shaft 60. In use, the user grasps the cradle member 22 with his hand(s) 58 and inserts the penile shaft 60 through the opening 29 with the concave side of the dorsal and ventral arm members facing toward the user's body. The device 22 is positioned at the base of the penile shaft 60 and folded so the dorsal arm member 26 is in contact with the dorsal side of the penile shaft 60 and the ventral arm member 28 is in contact with the ventral side of the penile shaft 60. The elastic strap 40 of the strap assembly 24 is then wrapped around the ventral arm member 28 and attached to the strap 42 on the dorsal arm member 26. The elastic strap 40 is placed under sufficient tension to retain the device 20 securely in place on the penile shaft 60. The flanges 27 of dorsal and ventral arm members 26, 28 are separated from one another when the cradle member 22 is positioned in the penile shaft 60 in its folded state.

As illustrated in Figure 6, when the device is in the folded, operative state on the penile shaft 60, the urethral occlusion pad 30 is positioned adjacent to the urethra 62 and imparts sufficient force to block the urethra so as to prevent involuntary loss of urine.

To urinate or remove the device 20, the elastic strap 40 is released from the tape 42 on the dorsal arm member 26 thereby enabling the cradle member 22 to be unfolded and slipped of the penile shaft 60.

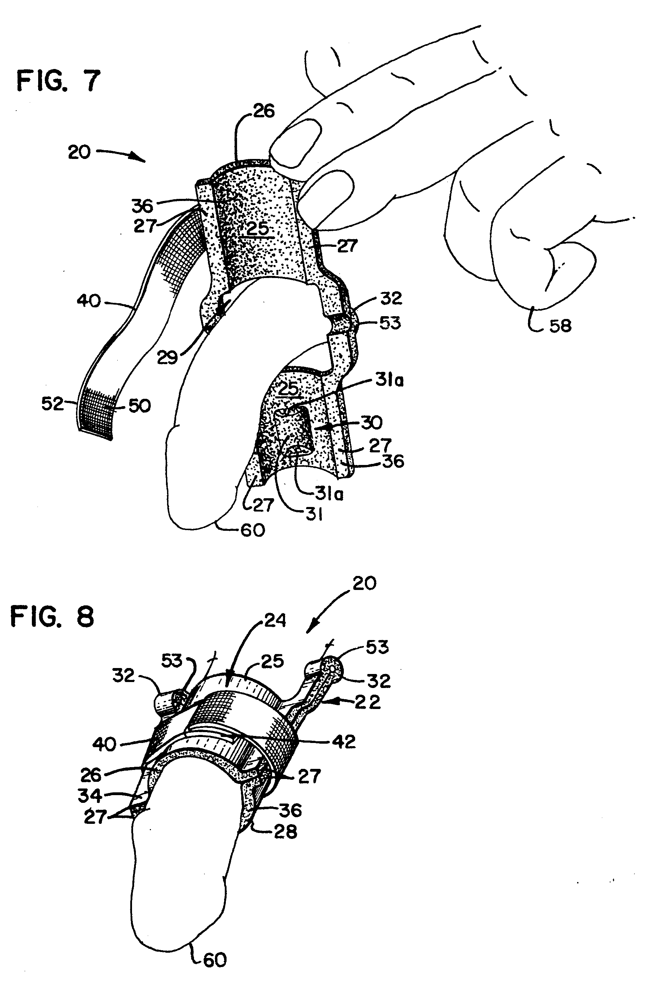

Figures 7 and 8 illustrate and alternative method of positioning the device 20 on the penile shaft 60. In this method, the device 20 is inserted onto the penile shaft 60 with the concave side of the dorsal and ventral arm members 26, 28 facing away from the user's body as generally illustrated in Figure 7. The cradle member 22 is then folded into its operative position as illustrated in Figure 8 with the hinges 32 being adjacent the base of the penile shaft 60 and the dorsal and ventral arm members 26, 28 extending away toward a distal end of the penile shaft 60.

It is to be understood, that even though numerous characteristics and advantages of the invention have been set forth in the foregoing description, together with details of the structure and function of the invention, the disclosure is illustrative only, and changes may be made in detail, especially in matters of shape, size, and arrangement of the parts within the principles of the invention to the full extent indicated by the broad general meaning of the terms in which the appended claims are expressed.

## Claims

1. An external male anti-incontinence device, comprising:
a) a cradle member (22) including a dorsal arm member (26) having a curvilinear surface (25) and a ventral arm member (28) having a curvilinear surface (25), the dorsal and ventral arm members (26,28) being in axial alignment with each other, an aperture (29) being defined intermediate of the dorsal and ventral arm members (26,28), the ventral arm member (28) including an integral pressure pad (30), the cradle member (22) further including integral hinges (32) interconnecting the dorsal and ventral arm members (26,28), the cradle memer (22) being hinged about an axis perpendicular to the axial alignment of the dorsal and ventral arm members (26,28) whereby the cradle member (22) can be folded over into a folded state so that the curvilinear surfaces (25) of the dorsal and ventral arm members (26,28) are facing one another with the pressure pad (30) projecting into a cavity formed between the facing curvilinear surfaces (25) of the dorsal and ventral arm members (26,28); and
b) strap assembly means (24) for releasably retaining the cradle member (22) in the folded state.

2. A device (20) according to claim 1, wherein the strap assembly (24) includes a length of elasticized tape (40) attached to the cradle member (22) proximate one end (44) and including a loop material (52) releasably attachable to hook material attached to the cradle member (22).

3. A device (20) according to claim 1, wherein the cradle member (22) comprises a polyolefin surface coating fused to closed-cell, chemically cross-linked polyethylene foam.

4. A device (20) according to claim 1, wherein the cradle members (26,28) are thermoformed.

5. A device (20) according to claim 1, wherein the cradle member (22) includes a top semi-rigid plastic layer attached to a bottom foam layer (36).

6. A device (20) according to claim 1, wherein the dorsal and ventral arm members (26, 28) include flange means (27) along their edges for limiting the force applied by the dorsal and ventral arm members (26,28).

7. A device (20) according to claim 6, wherein the strap assembly (24) includes an elastic member (40) retaining the dorsal and ventral arm members (26,28) in the folded state, the elastic member (40) enabling variable separation of the dorsal and ventral arm members (26,28).

8. A device (20) according to claim 1, wherein the hinges (32) are formed as arches (53) in the cradle member (22).

9. A device (20) according to claim 1, wherein the cradle member (22) is made of a one-piece construction.

10. A method for controlling male urinary incontinence using an external penile device (20) having an opening (29) for receipt of a penile shaft (60), dorsal and ventral members (26,28), and a urethral occlusion pad (30), the method comprising the steps of:
a) inserting the device (20) onto the penile shaft (60);
b) hinging the device (20) into a folded, operative state about an axis perpendicular to the penile shaft (60) so that the urethral occlusion pad (30) is blocking the urethra (62); and
c) releasably attaching the device (20) onto the penile shaft (60).

11. An external male anti-incontinence device (20), comprising:
a) cradle means (22) for positioning on a penile shaft (60), the cradle means (22) including a dorsal arm member (26) and a ventral arm member (28) in axial alignment with one another generally along a longitudinal axis of the cradle means (22), the cradle means (22) including an aperture (29) intermediate of the dorsal and ventral arm members (26,28), the cradle means (22) being hinged about an axis perpendicular to the longitudinal axis of the cradle means (22) so that the cradle means (22) is foldable between an unfolded state and a folded state wherein the dorsal and ventral arm members (26,28) are facing one another; and
b) means (40) releasably connecting the dorsal and ventral arm members (26,28) for retaining the cradle means (22) in the folded state.

## Patentansprüche

1. Eine äußerlich anzuwendende Anti-Inkontinenz-Vorrichtung für Männer mit:
a) einem Aufnahmeelement (22), das ein hinteres Armelement (26) mit einer gekrümmten Fläche (25) und ein vorderes Armelement (28) mit einer gekrümmten Fläche (25) aufweist, wobei das hintere und das vordere Armelement (26, 28) axial zueinander ausgerichtet sind, einer zwischen dem hinteren und dem vorderen Armelement (26, 28) ausgebildeten Öffnung (29), wobei das vordere Armelement (28) ein einstückiges Druckkissen (30) aufweist, das Aufnahmeelement (22) weiter einstückige Gelenke (32) aufweist, die das hintere und das vordere Armelement (26, 28) miteinander verbinden, das Aufnahmeelement (22) um eine Achse rechtwinklig zu der axialen Ausrichtung des vorderen und des hinteren Armelements (26, 28) klappbar ist, wodurch das Aufnahmeelement (22) in einen zusammengeklappten Zustand umgelegt werden kann, so daß die gekrümmten Flächen (25) des hinteren und des vorderen Armelements (26, 28) bei in die zwischen den aufeinander zuweisenden gekrümmten Flächen (25) des hinteren und des vorderen Armelements (26, 28) gebildete Höhlung ragenden Druckkissen (30) aufeinander zu weisen; und
b) Streifenbefestigungsmittel (24) zum lösbaren Halten des Aufnahmeelements (22) in dem zusammengeklappten Zustand.

2. Vorrichtung (20) nach Anspruch 1, wobei die Streifenbefestigung (24) ein Stück aus einem elastischen Band (40) aufweist, das an dem Aufnahmeelement (22) nahe dem einen Ende (44) angebracht ist und ein Schlaufenmaterial (52) aufweist, das lösbar an ein Klettmaterial, das an dem Aufnahmeelement (22) angeordnet ist, anbringbar ist.

3. Vorrichtung (20) nach Anspruch 1, wobei das Aufnahmeelement (22) eine Polyolefinflächenbeschichtung aufweist, die auf einen geschlossenzelligen, chemisch kreuzverbundenen Polyethylenschaum aufgeschweißt ist.

4. Vorrichtung (20) nach Anspruch 3, wobei die Aufnahmeelemente [richtig: Armelemente] (26, 28) thermogeformt sind.

5. Vorrichtung (20) nach Anspruch 1, wobei das Aufnahmeelement (22) eine obere, halbfeste Kunststoffschicht aufweist, die an einer Bodenschaumschicht (36) angebracht ist.

6. Vorrichtung (20) nach Anspruch 1, wobei das hintere und das vordere Armelement (26, 28) entlang ihrer Ränder Flanschmittel (27) zum Begrenzen der von dem hinteren und dem vorderen Armelement (26, 28) aufgebrachten Kraft aufweisen.

7. Vorrichtung (20) nach Anspruch 6, wobei die Streifenanordnung (24) ein elastisches Element (40) aufweist, das das hintere und das vordere Armelement (26, 28) in dem eingeklappten Zustand hält, wobei das elastische Element (40) einen variablen Abstand des hinteren und des vorderen Armelements (26, 28) voneinander ermöglicht.

8. Vorrichtung (20) nach Anspruch 1, wobei die Gelenke (32) als Bogen (53) in dem Aufnahmeelement (22) ausgebildet sind.

9. Vorrichtung (20) nach Anspruch 1, wobei das Aufnahmeelement (22) von einer einstückigen Konstruktion ist.

10. Verfahren zum Kontrollieren der Urininkontinenz des Mannes unter Verwendung einer außen an den Penis anzubringenden Vorrichtung (20), die mit einer Öffnung (29) zur Aufnahme eines Penisschafts (60), hinteren und vorderen Armelementen (26, 28) und einem Harnröhrenverschlußkissen (30) versehen ist, wobei das Verfahren die folgenden Schritte aufweist:
a) Aufsetzen der Vorrichtung (20) auf den Penisschaft (60);
b) Schwenken der Vorrichtung (20) in einen zusammengelegten Betriebszustand um eine Achse, die senkrecht zu dem Penisschaft (60) verläuft, so daß das Harnröhrenverschlußkissen (30) die Harnröhre (62) verschließt, und
c) lösbares Anbringen der Vorrichtung (20) auf den Penisschaft (60).

11. Eine äußerlich anzuwendende Anti-Inkontinenz-Vorrichtung (20) für den Mann, mit:
a) Aufnahmemitteln (22) zum Positionieren auf einem Penisschaft (60), wobei das Aufnahmemittel (22) ein hinteres Armelement (26) und ein vorderes Armelement (28) in axialer Ausrichtung miteinander im wesentlichen entlang einer Längsachse des Aufnahmeelements (22) aufweist und das Aufnahmeelement (22) mit einer Öffnung (29) zwischen dem hinteren und dem vorderen Armelement (26, 28) versehen ist, das Aufnahmeelement (22) um eine Achse zu der Längsachse des Aufnahmeelements (22) klappbar ist, so daß das Aufnahmeelement (22) zwischen einem gestreckten Zustand und einem umgelegten Zustand klappbar ist, in der das hintere und das vordere Armelement (26, 28) aufeinander zu weisen; und
b) Mitteln (40) zum lösbaren Verbinden des hinteren und des vorderen Armelements (26, 28) zum Halten des Aufnahmeelements (22) in dem umgelegten Zustand.

## Revendications

1. Dispositif contre l'incontinence chez l'homme comprenant :
a) un berceau (22) incluant un bras dorsal (26) présentant une surface curviligne (25) et un bras ventral (28) présentant une surface curviligne (25), les bras dorsal et ventral (26, 28) étant alignés axialement l'un par rapport à l'autre, une ouverture (29) étant définie entre les bras dorsal et ventral (26, 28), le bras ventral présentant un coussin de pression (30) intégré, le berceau (22) présentant des charnières intégrées reliant les bras dorsal et ventral (26, 28) entre eux, le berceau (22) étant articulé selon un axe perpendiculaire à l'axe d'alignement des bras dorsal et ventral (26, 28), dans lequel le berceau (22) peut être replié dans un état plié de façon telle que les surfaces curvilignes (25) des bras dorsal et ventral (26, 28) se fassent face l'une l'autre, le coussin de pression (30) faisant saillie dans la cavité formée entre les surfaces curvilignes (25) des bras dorsal et ventral (26, 28) ; et
b) des moyens d'assemblage par sangle (24) pour retenir de façon réversible le berceau (22) dans l'état plié.

2. Dispositif (20) selon la revendication 1, dans lequel l'assemblage par sangle (24) comprend une longueur de bande élastique (40) reliée au berceau (22) au niveau de l'une de ses extrémités (44) et incluant un matériau présentant des boucles (52) pouvant être relié de façon réversible à un matériau présentant des crochets fixé sur le berceau (22).

3. Dispositif (20) selon la revendication 1, dans lequel le berceau (22) comprend un revêtement de surface en polyoléfine lié par fusion à une mousse alvéolaire en polyéthylène réticulé par voie chimique.

4. Dispositif (20) selon la revendication 3, dans lesquels les éléments (26, 28) du berceau sont thermoformés.

5. Dispositif (20) selon la revendication 1, dans lequel le berceau (22) présente une couche supérieure en plastique semi-rigide liée à une couche de base (36) en mousse.

6. Dispositif (20) selon la revendication 1, dans lequel les bras dorsal et ventral (26, 28) incluent des articulations (27) sur leurs bords permettant de limiter la force appliquée par les bras dorsal et ventral (26, 28).

7. Dispositif (20) selon la revendication 6, dans lequel l'assemblage par sangle (24) comprend un élément élastique (40) retenant les bras dorsal et ventral (26, 28) dans l'état plié, l'élément élastique (40) permettant de réaliser une séparation variable entre les bras ventral et dorsal (26, 28).

8. Dispositif (20) selon la revendication 1, dans lequel les charnières (32) sont constituées par des arcs (53) formés dans le berceau (22).

9. Dispositif (20) selon la revendication 1, dans lequel le berceau (22) est constitué par un élément d'une seule pièce.

10. Procédé pour contrôler l'incontinence chez l'homme mettant en oeuvre un dispositif pénien externe (20) présentant une ouverture (29) pour recevoir une verge (60), des éléments dorsal et ventral (26, 28), et un coussin (30) d'occlusion urétrale, le procédé comprenant les étapes consistant à :
a) insérer le dispositif (20) sur la verge (60) ;
b) à articuler le dispositif (20) dans un état plié opérationnel selon un axe perpendiculaire à la verge (60) de façon telle que le coussin (30) d'occlusion urétrale bloque l'urètre (62) et,
c) à attacher de façon réversible le dispositif (20) sur la verge (60).

11. Dispositif externe contre l'incontinence chez l'homme comprenant :
a) un berceau (22) destiné à être positionné sur une verge (60), le berceau (22) incluant un bras dorsal (26) et un bras ventral (28) alignés axialement l'un par rapport à l'autre essentiellement selon l'axe longitudinal du berceau (22), le berceau (22) présentant une ouverture (29) située entre les bras dorsal et ventral (26, 28), le berceau (22) étant articulé selon un axe perpendiculaire à l'axe longitudinal du berceau (22) de sorte que le berceau (22) puisse être articulé entre un état non plié et un état plié dans lequel les bras dorsal et ventral (26, 28) se font face ; et
b) des moyens (40) pour connecter de façon réversible les bras dorsal et ventral (26, 28) afin de retenir le berceau (22) dans l'état plié.
